# EUROPEAN PATENT SPECIFICATION

(11) **EP 0 540 432 B1**
(45) Date of publication and mention of the grant of the patent: **28.06.1995**
(21) Application number: 92420370.6
(22) Date of filing: 22.10.1992
(51) Int. Cl.: G01N 33/569, G01N 33/68, C12P 21/08, C07K 16/16, G01N 33/577

(54) **Determination of adulteration of durum wheat**
Bestimmung der Verdorbenheit von Durumgetreides
Détermination d'aldultération de blé durum

(30) Priority: 26.10.1991 GB 9122775
(43) Date of publication of application: 05.05.1993
(73) Proprietor: RHONE-POULENC DIAGNOSTICS LIMITED, Glasgow G20 0SP, Scotland (GB)
(72) Inventor: Mackay, Eric Liddell, Rhone-Poulenc Diagnostics, Glasgow G1 1Y1 Scotland (GB); Stimson, William, Rhone-Poulenc Diagnostics Ltd., Glasgow G1 1Y1 Scotland (GB)
(74) Representative: Brachotte, Charles

(56) References cited:
- GENOME vol. 32, no. 6, 1989, MONTREAL CANADA pages 1096 - 1099 N. K. HOWES ET AL 'Screenig of durum wheats for pasta-making quality with monoclonal antibodies.'
- JOURNAL OF CEREAL SCIENCE vol. 12, 1990, NEW YORK NY USA pages 53 - 61 B.D. SULAIMAN ET AL. 'Proteins associated with the surface of wheat starch granules purified by centrifugation through Caesium Chloride.'

## Description

The present invention relates to the determination of non-durum grain in samples of durum wheat or durum-wheat containing products by immunological detection of a protein of recognised characteristics termed friabilin.

On a global basis, wheat is a major human food source. Many cultivars of wheat are known and their properties determine the uses to which the milled wheat flour is put. Wheats are generally categorised as either hard or soft. Soft wheats have a protein content of about 8 to 10% and are used for the production of cakes, biscuits and crackers. Hard wheat has a protein content of about 10 to 18% and is used primarily for bread making. In addition, durum wheat with a protein content of 10 to 16% is used for the production of pasta and semolina. Hard and soft wheat account for the vast majority of all wheat grown. Durum wheat is produced in relatively small quantities and is correspondingly valuable.

The quality of pasta produced from durum wheat flour is dependent on the quality of the flour. During growth, storage and transportation, accidental adulteration with non-durum wheats may occur. In addition, because of the high price it commands, it is common to find durum wheat adulterated with other types of wheat, or even other grains for profit. It is therefore desirable to be able to establish the durum wheat content of a sample of flour, in order to be able to assess its pasta-making properties.

Protein constitutes about 0.2% of wheat starch granules. The starch granule-associated proteins may be characterised by polyacrylamide gel electrophoresis in the presence of sodium dodecyl sulphate and 2-mercaptoethanol (SDS-PAGE). There are ten major polypeptides found in wheat starch granule protein. These include five surface proteins of relative molecular weight Mᵣ approximately equal to 5000, 8000, 14700, 19000 and 30000. There are also five integral proteins of molecular weights 59000, 77000, 86000, 95000, and 149000. The terms "surface protein" and "integral protein" relate to the temperature at which the proteins are extracted by water from the starch granules, the lower molecular weight surface proteins being extracted at 20°C, with the integral proteins requiring higher temperatures of 50°C and 100°C.

The amount of friabilin of relative molecular weight 14700 shows considerable variability between wheat varieties. The 14700 polypeptide band is strong in all soft wheats, but relatively weak in hard wheats. In durum wheats, the 14700 band is non-due to the lack of the corresponding gene in the durum wheat genome. A method of the screening samples of wheat for friabilin by gel electrophoresis is described in B. D. Sulaiman and W. R. Morrison, Journal of Cereal Science 12 (1990) pages 53-61.

Friabilin has been partially sequenced and the aminoterminal sequence of 36 aminoacids, one third of the molecule, has been determined to be

Ser-Gly-Pro-Trp-Met-Cys-Tyr-Pro-Gly-Gln-
Ala-Phe-Gln-Val-Pro-Ala-Leu-Pro-Ala-Cys-
Arg-Pro-Leu-Leu-Arg-Leu-Gln-Cys-Asn-Gly-
Ser-Gln-Val-Pro-Glu-Ala-

The present invention seeks to provide an assay for non-durum grain in durum wheat samples (including flour and prepared products) by detection of any friabilin content (which would be due to adulteration by non-durum grain).

Generally, the present invention relates to the use of a friabilin-specific monoclonal antibody for the detection of any friabilin content in a durum wheat sample by immunological reaction between the friabilin protein antigen and the antibody.

Specifically, one aspect of the present invention provides a method of assessing the quality of a grain sample containing durum wheat and possibly other non-durum grain to determine the content of said other non-durum grain, which comprises:
- providing a monoclonal antibody specific to friabilin protein;
- measuring the friabilin content of the sample by an immunoassay using the monoclonal antibody; and
- deducing the content of said other non-durum grain in the sample from the friabilin content of the sample.

The friabilin-specific antibody is an antibody which will bind to native or denatured friabilin protein with sufficient specificity to provide a useful assay. The antibody in particular will bind to heat-denatured friabilin and can thus be used on heat-treated grain or flour samples, for example a heat-denatured farinaceous food product. However the antibody will not bind to friabilin in which the disulphide bonds have been broken.

The friabilin-specific monoclonal antibody is made by conventional techniques. Typically, a mammal, such as a mouse, is immunised with friabilin or a friabilin-carrier protein conjugate, usually in the presence of adjuvant. Immunisation may be repeated until adequate antibody levels are provided. Antibody-producing spleen cells from the mammal are then fused with a substantially immortal cell line to prepare a hybridoma from which monoclonal antibodies may be isolated.

We have found an antigenically-effective region of friabilin to be adjacent one of the disulphide bridges. However, other antigenically-effective regions may also exist.

The antibody will generally be labelled in order to facilitate estimation of the immunological complex, for example by means of an enzyme label (commonly horseradish peroxidase or alkaline phosphatase), a radiolabel, a fluorescence label, or any other labelling system known in the art.

The immunoassay is conveniently conducted as an enzyme linked immunoassay (ELISA), wherein antibody conjugated to an enzyme (such as horseradish peroxidase or alkaline phosphatase) is reacted with the antigenic friabilin to produce a complex. The complex is estimated by adding a substrate for the enzyme, such as 3,3′,5,5′-tetramethylbenzidine, which is degraded to produce a coloured product. The coloured product generated is determined spectrophotometrically.

Alternatively, the complex can be estimated by reaction with a second monoclonal antibody which is specific to the first antibody. The second antibody may be for example an anti-mouse IgG monoclonal antibody . The second antibody may be labelled with a suitable tag, such as an enzyme, a radiolabel, a fluorescence label, colloidal gold or any other appropriate labelling system known in the art.

In an alternative embodiment, a plastic substrate, for example in the form of a rod or strip (e.g. a dipstick), may be provided with a surface coating which binds proteins from the wheat sample. This is then incubated with labelled antibody and the amount of complex estimated. If an enzyme labelled conjugate is used, the dipstick is further incubated with a substrate which produces a colour reaction, such that the degree of colour indicates the amount of friabilin present. Preferably the surface coating comprises nitrocellulose. It has been found that a nitrocellulose coating of thickness from 0.3 to 0.6 micromoters gives particularly good results in terms of colour clarity and sharpness.

In another embodiment, the invention provides a method for assessing the quality of a grain sample containing durum wheat and possibly other non-durum grain to determine the content of said other non-durum grain, which comprises:
- binding the wheat proteins in the sample to an affinity column (such as agarose coated with non-specific protein-binding agents);
- treating the column with non-specific proteins to block all the remaining reactive groups on the affinity substrate (for example milk proteins such as casein);
- treating the affinity substrate with a labelled monoclonal antibody (such as an antibody-enzyme conjugate) specific to friabilin protein;
- detecting the presence of the resulting friabilin-antibody conjugate using a precipitating substrate.

This method avoids the need for expensive spectrophotometric equipment. Examples of precipitating substrates include diaminobenzidine or preferably 4-chloro-1-naphthol.

The amount of friabilin present can also be determined by means of latex agglutination assay, wherein the monoclonal antibody is coupled to latex particles. Reaction with the friabilin antigen leads to agglutination of the latex particles. Similarly, cell agglutination assays can be conducted, wherein the antibody is coupled to sheep or human erythrocytes.

Thus, the invention also provides a friabilin-specific monoclonal antibody, and the antibody coupled to an enzyme label which allows quantitative assessment of the friabilin-antibody completed.

In a further aspect, the invention provides a kit for carrying out an ELISA determination of friabilin content in a durum wheat sample, which comprises an enzyme linked friabilin-specific monoclonal antibody.

In a further aspect, the invention provides a kit for determining friabilin content in a durum wheat sample, which comprises a plastic substrate having a surface coating which binds to proteins in the sample, and an enzyme linked friabilin-specific monoclonal antibody.

In a further aspect, the invention provides a second antibody, usually labelled, raised against the monoclonal antibody.

Embodiments of the invention will now be described by way of example only. In the Examples TWEEN 20 is a trade mark (polyoxyethylenesorbitan monolaurate).

### EXAMPLE 1

### Preparation of Monoclonal Antibody Conjugate F7F-HRP

The general procedure described in M.S. Islam and W.H. Stimson, Letters in Applied Microbiology 1987, Vol. 4, 85-89 was followed. The mice were immunised with a conjugate consisting of friabilin conjugated to keyhole limpet haemocyanin (KLH) by means of 1-ethyl-3-(3-dimethylaminopropyl) carbodiimide hydrochloride (EDC).

A group of 50 mice (NZB/BALB/c F1 hybrid females, 6-8 weeks old) were immunised with the conjugate (equivalent to 10 micrograms of friabilin) with Freunds Complete Adjuvant for the first injection, followed by subsequent injections of conjugate (5 micrograms friabilin) at four weekly intervals for a year. Ten mice were sacrificed and at the end of the year, one mouse showed antibody activity as determined by an ELISA test using friabilin-bovine serum albumen. This mouse is spleen cells were fused with myeloma cells by the general procedure referred to above. The monoclonal antibody produced was referred to as F7F.

The F7F monoclonal antibody was coupled to horseradish peroxidase (HRP) by means of a two-step sodium periodate procedure [Boorsma D.M. and Streefkerk J.G. (1979) J. Immunol. Methods, Vol. 30, 245] to produce the F7F-HRP conjugate.

### EXAMPLE 2

### Direct ELISA: Determination of Common Wheat in Durum Wheat Pasta

The following methodology was pursued.
1. Weigh out 50mg of finely ground dried pasta or flour.
2. Add 1ml of extraction buffer 0.25% w/v sodium dodecyl sulphate (SDS) in 50mM Tris/HCl buffer pH 7.4.
3. The sample of pasta can either be ground in extraction buffer using a mortar and pestle, or allowed to incubate for 1 hour at room temperature. In the case of flour, incubation is carried out for 10 minutes.
4. Centrifuge the extract of pasta to remove insoluble particles and dilute the supernatant 1/25 with 20mM Tris/HCl buffer containing 150mM NaCl pH 7.4 (TBS) to reduce the concentration of SDS to less than 0.02% to minimise interference with coating of the sample onto ELISA wells. If a centrifuge is not available, simply dilute the entire sample 1/25 to 25ml with TBS, mix well and allow the insoluble material to settle.
5. Coat 3 x 150 microlitre volumes into suitable ELISA plate wells.
6. Incubate the plate for 3 hours at 37°C or overnight at 4°C.
7. Wash plate wells six times with TBS buffer containing 0.05% TWEEN 20 surfactant.
8. Dry the plate on paper towels.
9. Add F7F-HRP (antibody-horseradish peroxide) conjugate 1/500 dilution in 10% v/v Normal Sheep Serum in TBS buffer containing 0.05% TWEEN 20, 150 microlitres per well.
10. Incubate the plate for 1.5 hours at 37°C to bind the antibody conjugate to any friabilin protein present.
11. Wash the plate wells six times with TBS containing 0.05% v/v TWEEN 20.
12. Dry the plate on tissues.
13. Add substrate prepared from 1/100 dilution stock, comprising
   3,3′,5,5′- tetramethylbenzidine (TMB), 6 mg/ml in dimethyl sulphoxide with 0.1M sodium acetate, and adjust pH to 6.0 with solid citric acid; add 5 microlitres 30% hydrogen peroxide to 25ml of TMB diluted solution at 150 microlitres per well.
14. Incubate for 15 minutes at room temperature to allow reaction between the HRP enzyme and the TMB substrate.
15. Stop reaction by addition of 75 microlitres of 10% sulphuric acid.
16. Read absorbances at 450nm on plate reader. The absorbance is proportional to the amount of friabilin/antibody-conjugate immunogenic complex present.

### RESULTS

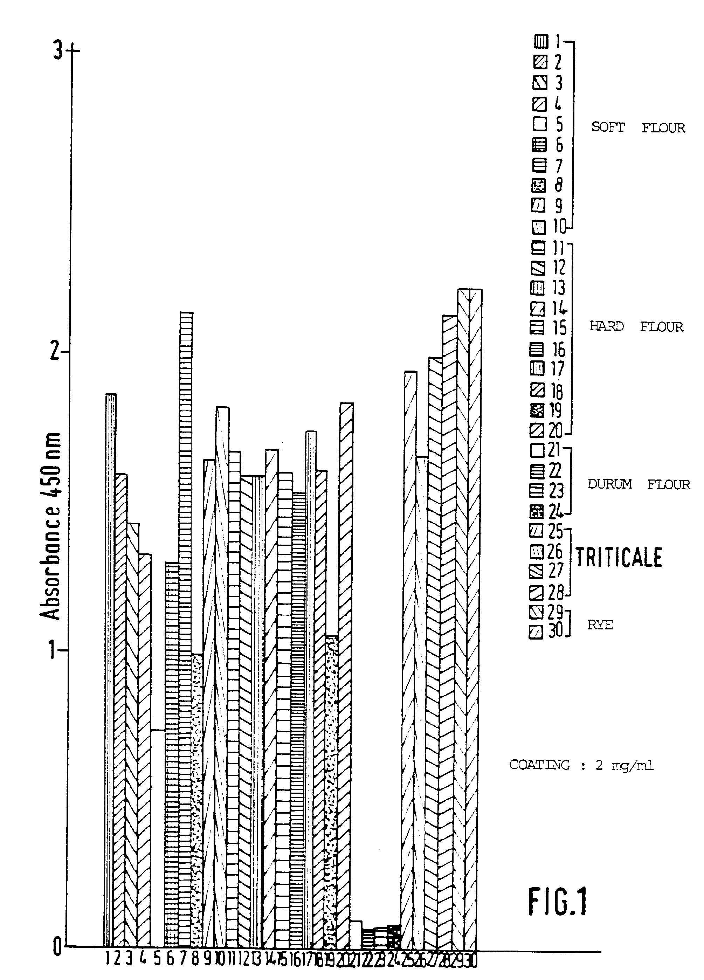

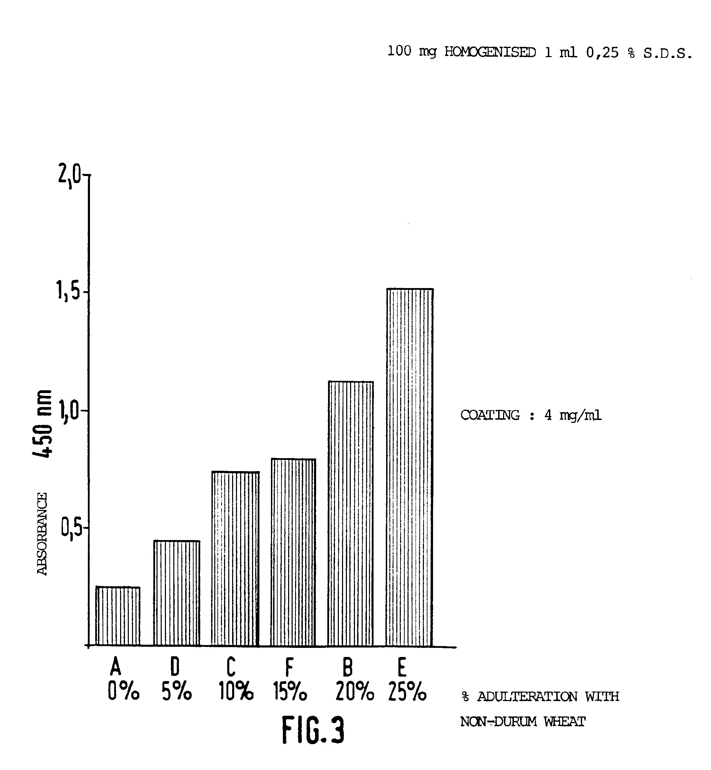

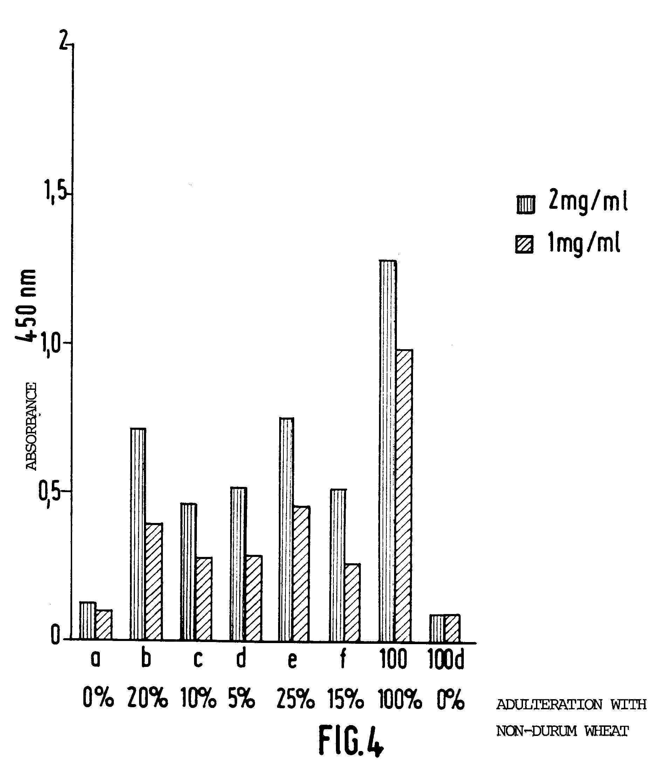

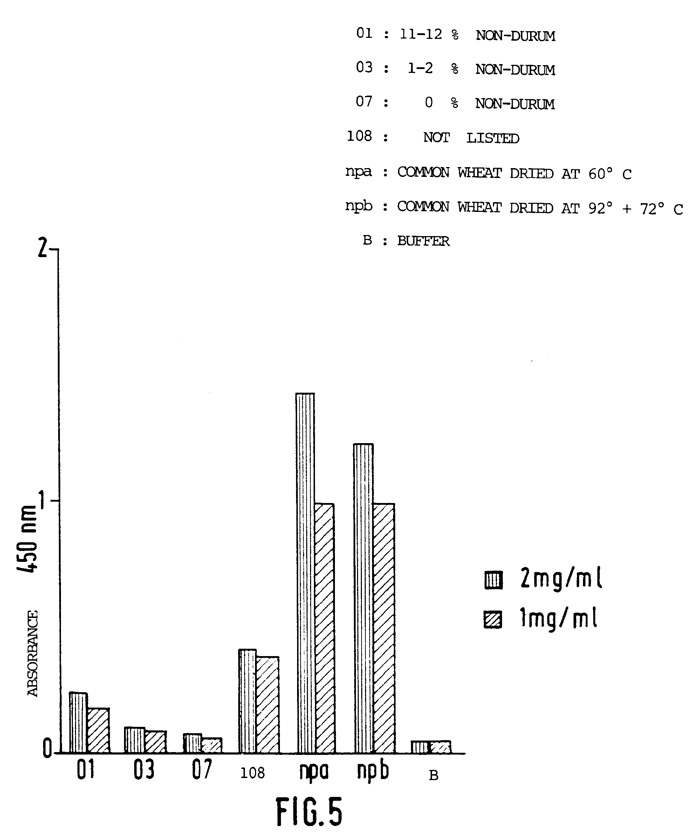

Figures 1 to 5 show results of the direct ELISA method carried out on various flour and pasta samples.
a) FIGURE 1 contains results of testing 30 flour samples supplied by Flour Milling and Baking Research Association (F.M.B.R.A.) with known Particle Size Index (P.S.I.) values as a parameter used to determine hardness/softness of wheat flours.
   Samples No. 1-10 are designated as Soft (P.S.I. >3.8)
   Samples No. 11-20 are designated as Hard (P.S.I. <3.8)
   Samples No. 21-24 are designated as durum samples (P.S.I. >2.0)
   Samples No. 25-28 are designated as crosses of Rye and Wheat (P.S.I. >3.3)
   Samples No. 29-30 are designated as Ryes (P.S.I. >5.9)
   These samples had previously consistently shown high friabilin values except the four durum samples which have always been negative.
   When selected samples from the above groupings were assessed using Western Blotting all show 15K protein detected using antibody F7F; except the durum samples which show a complete absence of any 15K basic protein.

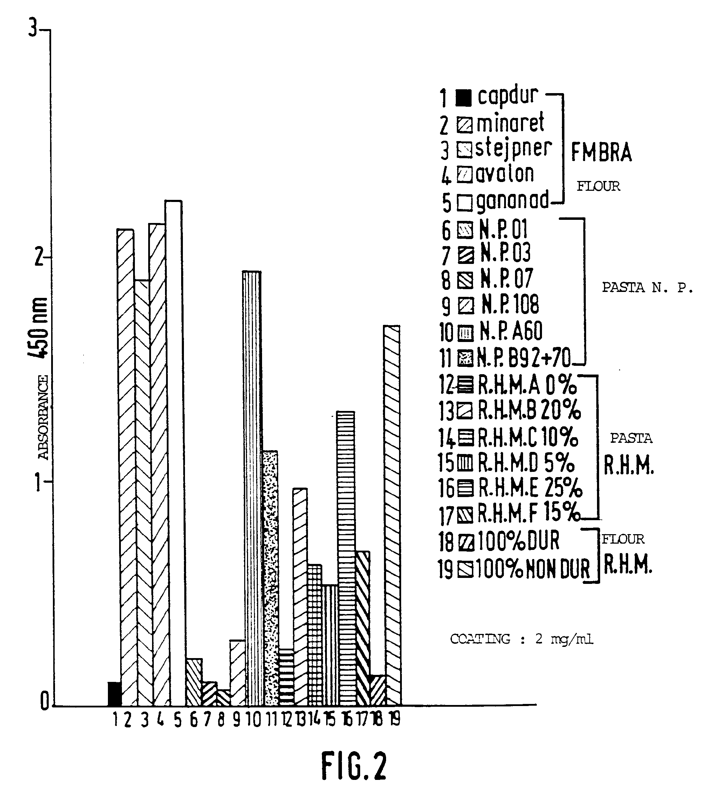

b) FIGURE 2 shows results for other samples which have been tested using the direct ELISA method.
   Samples 1-5 are flour samples from F.M.B.R.A. Sample 1 is a durum flour sample.
   Samples 6-11. This group of pasta samples was a standard set of samples from the Commission of the European Communities (Community Bureau of References). These samples were prepared from pasta samples which were dried at elevated temperatures of 92°C and 78°C. Present methods used to determine the presence of common wheats in pasta are based on the content of specific soluble proteins detected by gel electrophoresis. When pasta is prepared at elevated drying temperatures these proteins are denatured arid therefore these methods are proving unreliable. The present results show that antibody F7F detects the presence of friabilin in pasta standards which have been dried at elevated temperatures.
   Samples 12-19 These are further pasta standards from Rank Hovis McDougall (R.H.M.). These samples contain 0-25% adulteration with non-durum wheat. Results obtained appear to correlate well with expected values.
FIGURE 3 shows results obtained from pasta standards from R.H.M. with values plotted linear in relation to content of non-durum wheat.
FIGURE 4 shows the results from the samples of Figure 3 except that the extracts were diluted to 2mg/ml and 1mg/ml.
FIGURE 5 shows the results of the pasta samples of Figure 2, detailing the percentage adulteration by non-durum grain.

Sample 108 is believed to be 15% non-durum adulterated.

The same samples were run on a Western blot. The presence of a faint band at 15K in samples 01 and 108 was noted.

A Western blot was also performed to show total protein by staining using silver nitrate and ferric sulphate. This demonstrates the vast numbers of other proteins present in crude extracts from pasta or flour samples. Calculation of protein in coated samples of Figures 1 and 2 gives a value of approximately 200 micrograms/ml as assessed by Bradford's Protein assay.

The content of friabilin as a percentage of total flour protein was estimated to be less than 0.2% of total protein.

### EXAMPLE 3

### Dipstick Assay for Determination on non-Durum Wheats in pasta samples.

Due to the lengthy nature of the direct ELISA test (generally a minimum of 5 hours), other systems have been assessed using plastic sticks which have a surface of nitrocellulose to bind the pasta extract quickly; and subsequent incubation with F7F antibody horseradish peroxidase conjugate followed by incubation with insoluble peroxidase substrate 4-chloro-1-naphthol.

2.5 microlitres of filtered and centrifuged supernatant from a flour sample extract (200 mg/ml) of semolina (i.e. durum wheat) containing measured amounts of non-durum grain was spotted onto polypropylene strips coated with a 0.45 micrometers thick coating of nitrocellulose. The strips were washed and incubated in F7F-HRP for 15 minutes at room temperature, washed and incubated with 4-chloro-1-naphthol.

The degree of colour produced was found to be proportional to the amount of non-durum adulteration and could be read visually (Corresponding ELISA tests on the semolina standards also showed a linear response).

This method and considerably reduces the time-scale of the experiment to about 1 hour, although it is less quantitative in nature.

## Claims

1. The use of a friabilin-specific monoclonal antibody for the detection of any friabilin content in a durum wheat sample by immunological reaction between the friabilin protein antigen and the antibody.

2. A method of assessing the quality of a grain sample containing durum wheat and possibly other non-durum grain to determine the content of said other non-durum grain, which comprises:
- providing a monoclonal antibody specific to friabilin protein;
- measuring the friabilin content of the sample by an immunoassay using the monoclonal antibody; and
- deducing the content of said other non-durum grain contained in the sample from the friabilin content of the sample.

3. A method according to claim 2 wherein the monoclonal antibody is specific to friabilin protein having a molecular weight of approximately 14,700.

4. A method according to claim 2 or 3 wherein the grain sample is a flour sample.

5. A method according claim 3 or 4 wherein the grain sample is a heat-denatured farinaceous food product.

6. A method according to any one of claims 2 to 5 wherein the monoclonal antibody is enzyme-labelled and the immunoassay is carried out as an ELISA test.

7. A method according to claim 6 wherein the enzyme label is horseradish peroxidase.

8. A method according to any one of claims 2 to 7 wherein an extract of the grain sample is applied to a plastic substrate coated with a protein-binding material, and incubated with a second enzyme-labelled monoclonal antibody which is specific to the first antibody.

9. A method for assessing the quality of a grain sample containing durum wheat and possibly other non-durum grain to determine the content of said other non-durum grain, which comprises:
- binding the wheat proteins in the sample to an affinity column;
- treating the column with non-specific proteins to block all the remaining reactive groups on the affinity substrate;
- treating the affinity substrate with a labelled monoclonal antibody specific to friabilin protein;
- detecting the presence of the resulting friabilin-antibody conjugate using a precipitating substrate.

10. A method according to claim 8 wherein the monoclonal antibody is specific to friabilin protein having a molecular weight of approximately 14,700.

11. A method according to claim 9 or 10 wherein the precipitating substrate is 4-chloro-1-napthol.

12. Friabilin-specific monoclonal antibody.

13. A monoclonal antibody according to claim 12 which is specific to friabilin protein having a molecular weight of approximately 14,700.

14. Enzyme linked friabilin-specific monoclonal antibody.

15. A kit for carrying out an ELISA determination of friabilin content in a durum wheat sample, which comprises an enzyme linked friabilin-specific monoclonal antibody.

16. A kit according to claim 15 wherein the monclonal antibody is specific to friabilin protein having a molecular weight of approximately 14,700.

17. A kit according to claim 15 or 16 which comprises a plastic substrate having a surface coating which binds to proteins in the sample, and an enzyme linked friabilin-specific monoclonal antibody.

18. A kit according to claim 17 wherein the plasic substrate is in the form of a rod or strip.

19. A kit according to claim 17 or 18 wherein the surface coating is nitrocellulose.

20. A kit according to claim 19 wherein the nitrocellulose coating is 0.3 to 0.6 micrometers thick.

## Patentansprüche

1. Verwendung eines friabilinspezifischen monoklonalen Antikörpers zum Nachweis eines Gehalts an Friabilin in einer Durumweizenprobe durch immunologische Reaktion zwischen dem Friabilin-Protein-Antigen und dem Antikörper.

2. Verfahren zur Beurteilung der Qualität einer Getreideprobe, die Durumweizen und möglicherweise anderes, nicht aus Durumweizen bestehendes Getreide enthält, um den Gehalt an dem anderen, nicht aus Durumweizen bestehenden Getreide zu bestimmen, das folgende Schritte umfaßt:
- Bereitstellung eines für das Friabilin-Protein spezifischen monoklonalen Antikörpers,
- Mssung des Friabilin-Gehalts der Probe mit einem Immunoassay unter Verwendung des monoklonalen Antikörpers und
- Ableitung des Gehalts an dem in der Probe enthaltenen anderen, nicht aus Durumweizen bestehenden Getreide aus dem Friabilin-Gehalt der Probe.

3. Verfahren nach Anspruch 2, wobei der monoklonale Antikörper für ein Friabilin-Protein mit einem Molekulargewicht von etwa 14700 spezifisch ist.

4. Verfahren nach Anspruch 2 oder 3, wobei die Getreideprobe eine Mehlprobe ist.

5. Verfahren nach Anspruch 3 oder 4, wobei die Getreideprobe ein hitzedenaturiertes mehlhaltiges Nahrungsmittelprodukt ist.

6. Verfahren nach einem der Ansprüche 2 bis 5, bei dem der monoklonale Antikörper enzymmarkiert ist und der Immunoassay als ELISA-Test durchgeführt wird.

7. Verfahren nach Anspruch 6, wobei der Enzymmarker Meerrettichperoxidase ist.

8. Verfahren nach einem der Ansprüche 2 bis 7, bei dem ein Extrakt der Getreideprobe auf ein Kunststoffsubstrat aufgebracht wird, das mit einem proteinbindenden Material beschichtet ist, und mit einem zweiten enzymmarkierten monoklonalen Antikörper inkubiert wird, der für den ersten Antikörper spezifisch ist.

9. Verfahren zur Beurteilung der Qualität einer Getreideprobe, die Durumweizen und möglicherweise anderes, nicht aus Durumweizen bestehendes Getreide enthält, um den Gehalt an dem anderen, nicht aus Durumweizen bestehenden Getreide zu bestimmen, das folgende Schritte umfaßt:
- Bindung der Weizenproteine in der Probe an einer Affinitätssäule,
- Behandlung der Säule mit nichtspezifischen Proteinen, um sämtliche verbleibenden reaktiven Gruppen am Affinitätssubstrat zu blockieren,
- Behandlung des Affinitätssubstrats mit einem markierten monoklonalen Antikörper, der für das Friabilin-Protein spezifisch ist, und
- Erfassung des Vorliegens des resultierenden Friabilin-Antikörper-Konjugats unter Verwendung eines Ausfällsubstrats.

10. Verfahren nach Anspruch 8, bei dem der monoklonale Antikörper für ein Friabilin-Protein mit einem Molekulargewicht von etwa 14700 spezifisch ist.

11. Verfahren nach Anspruch 9 oder 10, wobei das Ausfällsubstrat 4-Chlor-1-naphthol ist.

12. Friabilinspezifischer monoklonaler Antikörper.

13. Monoklonaler Antikörper nach Anspruch 12, der spezifisch für ein Friabilin-Protein mit einem Molekulargewicht von etwa 14700 ist.

14. Enzymgebundener friabilinspezifischer monoklonaler Antikörper.

15. Kit zur Durchführung einer ELISA-Bestimmung des Friabilin-Gehalts in einer Durumweizenprobe, der einen enzymgebundenen friabilinspezifischen monoklonalen Antikörper enthält.

16. Kit nach Anspruch 15, bei dem der monoklonale Antikörper für ein Friabilin-Protein mit einem Molekulargewicht von etwa 14700 spezifisch ist.

17. Kit nach Anspruch 15 oder 16, der ein Kunststoffsubstrat mit einer Oberflächenbeschichtung, die an Proteine in der Probe bindet, und einen enzymgebundenen friabilinspezifischen monoklonalen Antikörper enthält.

18. Kit nach Anspruch 17, bei dem das Kunststoffsubstrat in Form eines Stäbchens oder Streifens vorliegt.

19. Kit nach Anspruch 17 oder 18, bei dem die Oberflächenbeschichtung aus Nitrocellulose besteht.

20. Kit nach Anspruch 19, bei dem die Nitrocellulose-Beschichtung 0,3 bis 0,6 »m dick ist.

## Revendications

1. Utilisation d'un anticorps monoclonal spécifique de la friabiline pour la détection d'une quantité quelconque de friabiline dans un échantillon de blé dur, par réaction immunologique entre l'antigène de la protéine friabiline et l'anticorps.

2. Une méthode d'évaluation de la qualité d'un échantillon de céréales contenant du blé dur et éventuellement d'autre céréales non dures afin d'évaluer la quantité des dites céréales non dures dans l'échantillon, caractérisée en ce qu'elle comprend les étapes suivantes :
- produire un anticorps monoclonal spécifique de la protéine friabiline ;
- mesurer la teneur en friabiline de l'échantillon par une analyse immunologique utilisant l'anticorps monoclonal ;
- déduire la quantité de céréales non dures contenue dans l'échantillon, de la teneur en friabiline de l'échantillon.

3. Une méthode selon la revendication 2, caractérisée en ce que l'anticorps monoclonal est spécifique de la protéine friabiline ayant un poids moléculaire d'environ 14700.

4. Une méthode selon la revendication 2 ou 3, caractérisée en ce que l'échantillon de céréales est un échantillon de farine.

5. Une méthode selon la revendication 3 ou 4, caractérisée en ce que l'échantillon de céréales est un produit alimentaire à base de farine dénaturé par la chaleur.

6. Une méthode selon l'une quelconque des revendications 2 à 5, caractérisée en ce que l'anticorps monoclonal est marqué par un enzyme et l'analyse immunologique est mise en oeuvre selon un test ELISA.

7. Une méthode selon la revendication 6, caractérisée en ce que le marqueur enzymatique est la peroxidase du raifort.

8. Une méthode selon l'une quelconque des revendications 2 à 7, caractérisée en ce que un extrait de l'échantillon de céréales est appliqué sur un substrat en matière plastique revêtu d'une substance fixant la protéine, et est mis en incubation avec un second anticorps monoclonal marqué par un enzyme spécifique du premier anticorps.

9. Une méthode d'évaluation de la qualité d'un échantillon de céréales contenant du blé dur et éventuellement d'autres céréales non dures, afin d'évaluer la quantité des dites céréales non dures dans l'échantillon, caractérisée en ce qu'elle comprend les étapes suivantes :
- fixer les protéines du blé de l'échantillon sur une colonne d'affinité;
- traiter la colonne avec des protéines non spécifiques pour bloquer tous les groupes réactifs restant sur le substrat d'affinité;
- traiter le substrat d'affinité avec un anticorps monoclonal marqué, spécifique de la protéine friabiline;
- détecter la présence du conjugué friabiline-anticorps en résultant en utilisant un substrat précipitant.

10. Une méthode selon la revendication 8, caractérisée en ce que l'anticorps monoclonal est spécifique de la protéine friabiline ayant un poids moléculaire d'environ 14.700.

11. Une méthode selon l'une des revendications 9 ou 10, caractérisée en ce que le substrat précipitant est le 4-chloro-1-naphtol.

12. Un anticorps monoclonal spécifique de la friabiline.

13. Un anticorps monoclonal selon la revendication 12, caractérisé en ce qu'il est spécifique de la protéine friabiline ayant un poids moléculaire d'environ 14.700.

14. Un anticorps monoclonal spécifique de la friabiline lié à un enzyme.

15. Un kit permettant la mise en oeuvre de la détermination, selon la méthode ELISA, de la teneur en friabiline d'un échantillon de blé dur, caractérisé en ce qu'il comprend un anticorps monoclonal spécifique de la friabiline lié à un enzyme.

16. Un kit selon la revendication 15, caractérisé en ce que l'anticorps monoclonal est spécifique de la protéine friabiline ayant un poids moléculaire d'environ 14.700.

17. Un kit selon la revendication 15 ou 16, caractérisé en ce qu'il comprend un substrat plastique ayant un revêtement de surface fixant les protéines de l'échantillon et un anticorps monoclonal spécifique de la friabiline lié a un enzyme.

18. Un kit selon la revendication 17, caractérisé en ce que le substrat en plastique se présentant sous la forme d'une baguette ou d'une bande.

19. Un kit selon la revendication 17 ou 18, caractérisé en ce que le revêtement de la surface est constitué de nitrocellulose.

20. Un kit selon la revendication 19, caractérisé en ce que le revêtement en nitrocellulose a une épaisseur de 0,3 à 0,6 microns.
